# EUROPEAN PATENT SPECIFICATION

(11) **EP 2 674 146 B1**
(45) Date of publication and mention of the grant of the patent: **31.08.2016**
(21) Application number: 12744167.3
(22) Date of filing: 31.01.2012
(51) Int. Cl.: A61K 8/49, A61K 8/06, A61K 8/891, A61K 8/894, A61Q 17/04

(54) **SUNSCREEN COSMETIC**
SONNENSCHUTZ-KOSMETIKUM
COSMÉTIQUE DE TYPE ÉCRAN SOLAIRE

(30) Priority: 07.02.2011 JP 2011023537
(43) Date of publication of application: 18.12.2013
(73) Proprietor: Shiseido Company, Ltd., Chuo-ku Tokyo 104-8010 (JP)
(72) Inventor: YAMAGUCHI, Kazuhiro, Yokohama-shi, Kanagawa 224-8558 (JP); NAGARE, Yuko, Yokohama-shi, Kanagawa 224-8558 (JP)
(74) Representative: Henkel, Breuer & Partner
(86) International application number: PCT/JP2012/052080
(87) International publication number: WO 2012/108298

(56) References cited:
- JP-A- 2007 145 722
- JP-A- 2008 143 823
- JP-A- 2010 532 812
- DATABASE WPI Week 201036 Thomson Scientific, London, GB; AN 2010-F40660 XP002729377, & JP 2010 111625 A (KAO CORP) 20 May 2010 (2010-05-20)
- DATABASE WPI Week 200958 Thomson Scientific, London, GB; AN 2009-M93539 XP002729378, & JP 2009 191033 A (KOSE KK) 27 August 2009 (2009-08-27)
- DATABASE WPI Week 201045 Thomson Scientific, London, GB; AN 2010-H40837 XP002729379, & WO 2010/070867 A1 (SHISEIDO CO LTD) 24 June 2010 (2010-06-24)

## Description

### Technical Field

The present invention relates to an oil-in-water emulsion composition, and more specifically to an oil-in-water emulsion composition that contains an oil-soluble ultraviolet absorbent in the outer phase (water phase) to exhibit stable and good ultraviolet protection ability.

### Background Art

Sunscreen cosmetics are intended to cut off ultraviolet rays in sunlight to protect the skin from adverse influence of ultraviolet rays. Examples of sunscreen cosmetic bases include emulsion type, lotion type, and oil type. Among these, oil-in-water emulsion type is widely used because it has a moist feel on use and can be formulated into a broad range of products including low SPF to high SPF products (Non Patent Document 1).

Meanwhile, the ultraviolet absorbents added to sunscreen cosmetics are classified into oil-soluble ones and water-soluble ones. To achieve high protection ability by absorption of ultraviolet rays in the UVA region (wavelengths from 320 to 400 nm) and the UVB region (wavelengths from 290 to 320 nm), well balanced amounts of a UVB absorbent and a UVA absorbent have to be added.

However, UVA absorbents, which absorb the UVA wavelength longer than the UVB wavelength, tend to have a conjugated bond longer than that of UVB absorbents to temporarily absorb energy. Thus, UVA absorbents generally have larger molecular weight than UVB absorbents, and accordingly, often become poorly soluble in oils. Dissolution of such absorbents requires addition of highly polar oil in a large amount. This has caused loss of a moist feel on use specific to oil-in-water emulsions or has resulted in a stability problem such as precipitation of the ultraviolet absorbent in the oil phase at low temperatures.

Patent Document 1 suggests addition of a specific polyether-modified silicone and hydrophobization of surface of a metal oxide powder to stably add diethylamino hydroxybenzoyl hexyl benzoate, which is an ultraviolet absorbent with poor solubility, and the metal oxide powder, which is an ultraviolet scattering agent. The document states that the resulting cosmetic composition has an excellent feeling on use and water resistance.

Patent Document 2 discloses that adding partially-crosslinked polyether-modified organopolysiloxane, organic titanate-treated zinc oxide or titanium dioxide particulates, a silicone oil, a specific oil component, and an inulin fatty acid ester in combination provides a sunscreen cosmetic that exhibits excellent dispersion and emulsion stability as well as has a good feel on use.

The polyether-modified silicones (crosslinked type) used in Patent Documents 1 and 2, which are added also in the sunscreen cosmetics described in Patent Documents 3 and 4, is suggested to contribute to enhancing the dispersion of titanium dioxide or the stability of the emulsion.

However, the cosmetics described in Patent Documents 1 to 3 use powders such as titanium dioxide as essential components, which are inevitably accompanied by excessive white masking and a reduced feeling on use. Examples of co-addition of crosslinked polyether-modified silicone into a formulation containing a poorly soluble ultraviolet absorbent in the water phase have been neither disclosed nor suggested.

### Prior Art Publications

### Patent Document

Patent Document 1: JP-A 2010-111625
Patent Document 2: JP-A 2009-191033
Patent Document 3: JP-A 2010-143852
Patent Document 4: JP-A 2009-524644

### Non Patent Document

Non Patent Document 1: "New Cosmetic Science, 2nd edition," edited by Takeo Mitsui, Nanzando Co., Ltd., 2001, pp. 497-504

### Summary of Invention

### Problems to be solved by the Invention

Accordingly, an object of the present invention is to provide a sunscreen cosmetic composition that effectively protects from both UVA and UVB, has moist and cool texture well as excellent stability, and is able to form a film having a good ultraviolet protection effect when applied to skin.

### Means for solving the Problems

To solve the problems described above, the present invention provides an oil-in-water sunscreen cosmetic composition, comprising:
(a) an aqueous dispersion having composite particles of bis-ethylhexyloxyphenol methoxyphenyl triazine and an organic polymer dispersed therein, wherein said composite particles are obtainable by emulsion-polymerization of a mixture of bis-ethylhexyloxyphenol methoxyphenyl triazine and an organic monomer having an ethylenically unsaturated bond dispersed in water;
(b) 0.01 to 5% by mass of one or two or more selected from crosslinked polyether-modified silicones and crosslinked alkyl-polyether-modified silicones;
(c) 0.01 to 30% by mass of a dimethicone having a viscosity of 10 mPa·s or less; and
(d) 0.01 to 80% by mass of water,
wherein the amount of bis-ethylhexyloxyphenol methoxyphenyl triazine in the cosmetic composition is 0.01 to 5% by mass.

### Effects of the Invention

The sunscreen cosmetic composition of the present invention has been able to improve the stability of the system by adding a poorly oil-soluble ultraviolet absorbent in the water phase. Additionally, the cosmetic composition also has a beneficial effect of enhancing ultraviolet protection ability relative to a case in which the ultraviolet absorbent is added in the oil phase. The system of the cosmetic composition has further been stabilized by addition of agar microgel. Therefore, the cosmetic composition of the present invention is particularly suitable to use as a sunscreen cosmetic that has a moist feel on use specific to an oil-in-water emulsion as well as excellent ultraviolet protection ability.

### Brief Description of Drawing

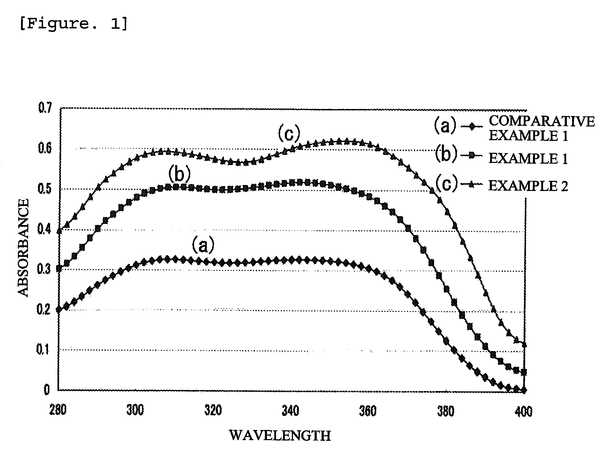

[Figure 1] Figure 1 is a graph showing the ultraviolet absorption spectra of the compositions according to Examples 1, 2 and Comparative Example 1.

### Modes for carrying out the Invention

The sunscreen cosmetic composition of the present invention is characterized in that it comprises an aqueous dispersion of an oil-soluble ultraviolet absorbent (component a) in the water phase.

The oil-soluble ultraviolet absorbent is insoluble in water and poorly soluble in oil. However, absorbents substantially insoluble in oil, such as methylene bis-benzotriazole tetramethyl butylphenol are not contained. When an oil-in-water emulsion composition, which has been prepared using an aqueous dispersion of an oil-insoluble ultraviolet absorbent, is applied to the skin, the resultant skin may look unnaturally whitish.

Poorly soluble ultraviolet absorbents include those described in Patent Document 1, specifically, such as benzophenone derivatives and triazine derivatives. According to the invention, the oil-soluble ultraviolet absorbent is a triazine derivative, namely 2,4-bis-{[4-(2-ethylhexyloxy)-2-hydroxy]phenyl}-6-(4-methoxyphenyl)-(1,3,5)-triazine (hereinafter referred to as "bis-ethylhexyloxyphenol methoxyphenyl triazine"). This bis-ethylhexyloxyphenol methoxyphenyl triazine is commercially available from BASF SE under the trade name Tinosorb S, and the commercially available product can be used.

The aqueous dispersion of the oil-soluble ultraviolet absorbent is an aqueous dispersion of composite particles of an oil-soluble ultraviolet absorbent and an organic polymer. When the water phase containing the aqueous dispersion coexists with an oil phase, making of the composite particles suppress dissolution of the oil-soluble ultraviolet absorbent from the water phase into the oil phase.

The aqueous dispersion of composite particles of an oil-soluble ultraviolet absorber and an organic polymer can be prepared, for example, in accordance with the method described in WO2009/007264. In short, a mixture of the ultraviolet absorbent and an organic monomer dispersed in water is emulsion-polymerized to provide an aqueous dispersion having composite particles of the ultraviolet absorbent and the organic polymer dispersed therein.

The organic monomer is a monomer having an ethylenically unsaturated bond, for example, acrylic acid, methacrylic acid, alkyl acrylate, alkyl methacrylate, a styrene monomer, or a nylon monomer.

As an aqueous dispersion of such composite particles, a commercially available product from BASF SE under the trade name Tinosorb S aqua can be used. Tinosorb S aqua contains composite particles of bis-ethylhexyloxyphenol methoxyphenyl triazine and polymethylmethacrylate (PMMA) dispersed in water. The contents of bis-ethylhexyloxyphenol methoxyphenyl triazine and PMMA are 20% by mass and 19% by mass, respectively.

The amount of the oil-soluble ultraviolet absorbent to be added in the cosmetic of the present invention is 0.01 to 5% by mass, preferably 0.01 to 3% by mass, on a dry mass basis. If the amount is less than 0.01% by mass, sufficient ultraviolet absorption ability cannot be achieved; whereas, if the amount is more than 5% by mass, a problem in texture such as stickiness tends to arise.

If the amount is converted to an amount of a water dispersion containing 20% by mass of an ultraviolet absorbent (component a) to be added, for example, the aqueous dispersion will be added at 0.05 to 25% by mass, preferably at 0.05 to 15% by mass.

The cosmetic composition of the present invention contains one or two or more selected from crosslinked polyether-modified silicones and crosslinked alkyl polyether-modified silicones (component b) as essential components.

Examples of crosslinked polyether silicones (also referred to as polyether-modified crosslinked silicones) are polyether-modified crosslinked silicones such as (dimethicone/(PEG-10/15)) crosspolymer. Crosslinked alkyl-polyether-modified silicones, which are products obtained by further alkyl modification of crosslinked polyether-modified silicones (alkyl co-modification type), include, for example, (PEG-10/lauryl dimethicone) crosspolymer and (PEG-15/lauryl dimethicone) crosspolymer.

As these crosslinked polyether-modified silicones and crosslinked alkyl polyether-modified silicones, commercially available products may be used. Examples of these silicones include gelled dispersions kneaded with various cosmetic oils (for example, dimethicone, mineral oils, trioctanoin, and squalane), which are marketed from Shin-Etsu Silicone under product numbers KSG21, KSG210, KSG240, KSG310, KSG330, and KSG340.

The amount of one or two or more selected from crosslinked polyether-modified silicones and crosslinked alkyl polyether-modified silicones (component b) to be added in the cosmetic of the present invention is 0.01 to 5% by mass, preferably 0.01 to 3% by mass. If the amount to be added is less than 0.01% by mass, a stable emulsion is difficult to obtain; whereas, if the amount is more than 5% by mass, a problem in texture such as stickiness may arise.

The cosmetic composition of the present invention also contains a low-viscosity silicone which is a dimethicone with a viscosity of 10 mPa·s or less, preferably 5 mPa·s or less, such as decamethylcyclopentasiloxane. Addition of the low-viscosity silicone oil can reduce stickiness and squeakiness on application.

The amount of the low-viscosity silicone oil in the cosmetic composition of the present invention is 0.01 to 30% by mass, preferably 0.01 to 15% by mass, and more preferably 0.01 to 10% by mass. If the amount is more than 30% by mass, a stability problem such as separation or a problem in texture such as loss of a moist feeling on use may arise.

The cosmetic of the present invention further contains water (component d) as an essential component.

The amount of water to be added in the cosmetic composition of the present invention is 0.01 to 80% by mass, preferably 0.01 to 50% by mass, and more preferably 0.01 to 30% by mass. If the amount is more than 80% by mass, a stability problem such as separation or a problem in texture such as loss of a moist feeling on use may arise.

Furthermore, the cosmetic composition of the present invention preferably has methylene bisbenzotriazolyl tetramethylbutylphenol (component e) added in the water phase. Addition of methylene bisbenzotriazolyl tetramethylbutylphenol further enhances the ultraviolet protection effect.

Methylene bisbenzotriazolyl tetramethylbutylphenol, which is insoluble in water, is preferably dispersed in the water phase before admixture. For example, an aqueous dispersion of methylene bisbenzotriazolyl tetramethylbutylphenol, commercially available from BASF SE under the trade name Tinosorb M, can be used.

The amount of methylene bisbenzotriazolyl tetramethylbutylphenol to be added in the cosmetic of the present invention is 20% by mass or less, preferably 10% by mass or less, and more preferably 1 to 9% by mass. If the amount is more than 20% by mass, a problem such as squeakiness on application may arise.

Other ultraviolet absorbents may be further added to the compositions of the present invention, in addition to the aqueous dispersion of the oil-soluble ultraviolet absorbent (component a) and methylene bisbenzotriazolyl tetramethylbutylphenol (component e) added in the water phase.

Other ultraviolet absorbents, which are oil soluble, preferably dissolve into the oil phase and absorb ultraviolet radiation synergistically with the ultraviolet absorbent in the water phase.

Such ultraviolet absorbents include, but not particularly limited to, methoxy cinnamic acid derivatives, diphenyl acrylic acid derivatives, salicylic acid derivatives, para-aminobenzoic acid derivatives, triazine derivatives, benzophenone derivatives, benzal malonate derivatives, anthranilic derivatives, imidazoline derivatives, 4,4-diaryl butadiene derivatives, and phenylbenzimidazole derivatives. Specific examples include 2-ethylhexyl p-methoxycinnamate, homosalate, octyl salicylate, oxybenzone, 4-t-butyl-4'-methoxydibenzoylmethane, octyltriazone, bis-ethylhexylphenol methoxyphenyl triazine, 2-hydroxy-4-methoxybenzophenone, dihydroxy dimethoxybenzophenone, dihydroxybenzophenone, tetrahydroxybenzophenone, diethylamino hydroxybenzoyl hexyl benzoate, 2-cyano-3,3-diphenylacrylic acid-2'-ethylhexyl ester, polysilicone-15, and drometrizole polysiloxane.

The cosmetic composition of the present invention may also contain powders of metal oxides that serve as ultraviolet scattering agents, including titanium dioxide and zinc oxide.

However, the sunscreen cosmetic composition of the present invention, which contains essential components such as bis-ethylhexyloxyphenol methoxyphenyl triazine in the water phase and optionally contains methylene bisbenzotriazolyl tetramethylbutylphenol or other ultraviolet absorbents, has good ultraviolet protection ability throughout the UVA to UVB regions. Thus, the cosmetic composition of the present invention may not contain ultraviolet scattering agents, which may be responsible for excessive white masking on application.

In addition to the components described above, the cosmetic composition of the present invention can contain other components usually used in compositions for external use such as cosmetics to the extent where the intended effect of the present invention is not substantially prevented.

The compositions of the present invention can be prepared by separately mixing components constituting the oil phase and components constituting the water phase followed by adding the oil phase into the water phase for emulsification.

The composition of the present invention has a moist feel on use intrinsic to an oil-in-water emulsion, provides excellent stability at low and high temperatures, and exhibits excellent ultraviolet protection ability. As such, the composition is particularly suitable to use as a sunscreen cosmetic composition of oil-in-water emulsion type.

### Examples

Hereinbelow, the present invention is described in greater detail in view of the specific examples, but the present invention is not limited to the Examples given below. Further, the amounts mentioned in the following Examples indicate % by mass, unless specifically described otherwise.

Oil-in-water emulsion compositions with the composition shown in the following Table 1 were prepared. Specifically, water phase components and oil phase components were separately heated to 70°C to be completely dissolved, and then, the oil phase was added to the water phase for emulsification by an emulsifier to obtain compositions of Examples.

To a surface of a PMMA film (5 cm × 5 cm), 18.87 µL of a sample of each of the compositions of Examples 1, 2, and Comparative Example 1 was uniformly applied at a ratio of 0.75 mg/cm². After left for 15 minutes, each sample was measured for absorbance by using a spectrophotometer (U-4100: manufactured by Hitachi, Ltd.). The results are shown in Figure 1.

**[Table 1]**

| | Comparative Example 1 | Example 1 | Example 2 |
|---|---|---|---|
| Methylene bisbenzotriazolyl tetramethylbutylphenol^{*1)} | - | - | 6 |
| Bis-ethylhexyloxyphenol methoxyphenyl triazine^{*2)} (dissolved in the oil phase before addition) | 3 | - | - |
| Bis-ethylhexyloxyphenol methoxyphenyl triazine/PMMA composite^{*3)} | - | 15 | 15 |
| 1,3-Butylene glycol | 3 | 3 | 3 |
| Phenoxyethanol | 0.5 | 0.5 | 0.5 |
| Ion-exchanged water | Balance | Balance | Balance |
| Ethyl alcohol | 3 | 3 | 3 |
| EDTA·3Na2H2O | 0.2 | 0.2 | 0.2 |
| Sodium chloride | 1 | 1 | 1 |
| Glycerin | 3 | 3 | 3 |
| Decamethylcyclopentasiloxane | 15 | 15 | 15 |
| Isododecane | 20 | 20 | 20 |
| Dimethicone (viscosity: 2 mPa·s) | 15 | 15 | 15 |
| Triethylhexanoin | 10 | - | - |
| Silicone gel^{*4)} | 5 | 5 | 5 |

| | | | |
|---|---|---|---|
| *1) Tinosorb M (manufactured by BASF SE) *2) Tinosorb S (manufactured by BASF SE): bis-ethylhexyloxyphenol methoxyphenyl triazine *3) Tinosorb S aqua (20% aqueous dispersion: manufactured by BASF SE) *4) KSG-210 (manufactured by Shin-Etsu Chemical Co., Ltd.): silicone gel comprising 70-80% of dimethicone and 20-30% of a crosslinked polyether-modified silicone ((dimethicone/(PEG-10/15)) crosspolymer) | | | |

As shown in Figure 1, Example 1, in which bis-ethylhexyloxyphenol methoxyphenyl triazine was added to the water phase, has enhanced the ultraviolet protection effect relative to Comparative Example 1, in which bis-ethylhexyloxyphenol methoxyphenyl triazine was dissolved in the oil phase. Moreover, Example 2, in which methylene bisbenzotriazolyl tetramethylbutylphenol was added, has further enhanced the ultraviolet protection effect.

Oil-in-water emulsion compositions with the composition shown in the following Tables 2 and 3 were prepared. Specifically, water phase components and oil phase components were separately heated to 70°C to be completely dissolved, and then, the oil phase was added to the water phase for emulsification by an emulsifier to obtain compositions of Examples.

As described above, each composition was evaluated for absorbance at the wavelength of 310 nm, stability at room temperature, and usability such as excessive whiteness and stickiness. Method and criteria of evaluation are as follows. The results are shown in both Tables 2 and 3.

### 1. Room temperature stability

After left at room temperature, the compositions were evaluated by visual observation.
○: No precipitation of ultraviolet absorbent was observed.
×: Precipitation of ultraviolet absorbent was observed.

### 2. Feeling on use (excessive white masking)

Female panelists (N = 20) were asked to apply each composition on their skin and to evaluate the presence or absence of excessive white masking based on the following criteria.
○: Sixteen or more panelists found no excessive white masking.
Δ: Six to fifteen panelists found no excessive white masking.
×: Five or fewer panelists found no excessive white masking.

### 3. Feeling on use (stickiness)

Female panelists (N = 20) were asked to apply each composition on their skin and to evaluate stickiness when the composition blended into their skin based on the following criteria.
○: Sixteen or more panelists found no stickiness.
Δ: Six to fifteen panelists found no stickiness.
×: Five or fewer panelists found no stickiness.

**[Table 2]**

| | Comparative Example | | | |
|---|---|---|---|---|
| | 2 | 3 | 4 | 5 |
| Decamethylcyclopentasiloxane (D5) | 15 | 15 | 15 | 15 |
| Lauryl PEG-9 polydimethylsiloxyethyl dimethicone^{*1)} | - | - | - | - |
| Silicone gel^{*2)} | 7 | 7 | 7 | 7 |
| Caprylyl methicone | 5 | 5 | 5 | 5 |
| Isododecane | 5 | 5 | 5 | 5 |
| Ethylhexyl methoxycinnamate^{*3)} | 3.5 | 9 | 9 | 7 |
| Diethylamino hydroxybenzoyl hexyl benzoate^{*4)} | - | 1 | 1 | - |
| Bis-ethylhexyloxyphenol methoxyphenyl triazine^{*5)} | - | - | - | 3 |
| Bis-ethylhexyloxyphenol methoxyphenyl triazine/PMMA composite^{*6)} | - | - | - | - |
| Phenylbenzimidazole sulfonic acid | 3.5 | - | - | - |
| Methylene bisbenzotriazolyl tetramethylbutylphenol^{*7}) | - | - | - | - |
| (Vinyl dimethicone/methicone silsesquioxane) crosspolymer | 1.5 | 1.5 | 1.5 | 1.5 |
| Particulate titanium dioxide | 2 | - | - | - |
| Hydrophobized particulate zinc oxide | - | - | 5 | - |
| Ion-exchanged water | Balance | Balance | Balance | Balance |
| Triethanolamine | 2.5 | - | - | - |
| EDTA·3Na2H2O | 0.2 | 0.2 | 0.2 | 0.2 |
| Sodium chloride | 0.5 | 0.5 | 0.5 | 0.5 |
| 1,3-Butylene glycol | 3 | 3 | 3 | 3 |
| Ethyl alcohol | 3 | 3 | 3 | 3 |
| Phenoxyethanol | 0.5 | 0.5 | 0.5 | 0.5 |
| Glycerin | 3 | 3 | 3 | 3 |
| Ultraviolet protection effect (absorbance at 310 nm) | 1.09 | 1.18 | 1.12 | 1.27 |
| Room temperature stability (precipitation) | ○ | ○ | ○ | × |
| Excessive whiteness | Δ | ○ | Δ | ○ |
| Stickiness | ○ | Δ | ○ | Δ |

| | | | | |
|---|---|---|---|---|
| *1) KF6038 (manufactured by Shin-Etsu Chemical Co., Ltd.) *2) KSG-210 (manufactured by Shin-Etsu Chemical Co., Ltd.) *3) Parsol MCX *4) Uvinul A plus *5) Tinosorb S (manufactured by BASF SE) *6) Tinosorb S aqua (20% aqueous dispersion: manufactured by BASF SE) *7) Tinosorb M (manufactured by BASF SE) | | | | |

**[Table 3]**

| | Example | | |
|---|---|---|---|
| | 3 | 4 | 5 |
| Decamethylcyclopentasiloxane (D5) | 15 | 15 | 15 |
| Lauryl PEG-9 polvdimethylsiloxyethyl dimethicone^{*1)} | - | - | 0.2 |
| Silicone gel^{*2)} | 7 | 7 | 7 |
| Caprylyl methicone | 5 | 5 | 5 |
| Isododecane | 5 | 5 | 5 |
| Ethylhexyl methoxycinnamate^{*3)} | 7 | 7 | 7 |
| Diethylamino hydroxybenzoyl hexyl benzoate^{*4)} | - | - | - |
| Bis-ethylhexyloxyphenol methoxyphenyl triazine^{*5)} | - | - | - |
| Bis-ethylhexyloxyphenol methoxyphenyl triazine/PMMA composite^{*6)} | 15 | 15 | 9.5 |
| Phenylbenzimidazole sulfonic acid | - | - | 3 |
| Methylene bisbenzotriazolyl tetramethyl butylphenol ^{*7)} | - | - | 6 |
| (Vinyl dimethicone/methicone silsesquioxane) crosspolymer | 1.5 | 1.5 | 1.5 |
| Particulate titanium dioxide | - | - | - |
| Hydrophobized particulate zinc oxide | - | 5 | 5 |
| Ion-exchanged water | Balance | Balance | Balance |
| Triethanolamine | - | - | 2 |
| EDTA·3Na2H2O | 0.2 | 0.2 | 0.2 |
| Sodium chloride | 0.5 | 0.5 | 0.5 |
| 1,3-Butylene glycol | 3 | 3 | 3 |
| Ethyl alcohol | 3 | 3 | 3 |
| Phenoxyethanol | 0.5 | 0.5 | 0.5 |
| Glycerin | 3 | 3 | 3 |
| Ultraviolet protection effect (absorbance at 310 nm) | 1.64 | 1.72 | 1.71 |
| Room temperature stability (precipitation) | ○ | ○ | ○ |
| Excessive whiteness | ○ | Δ | Δ |
| Stickiness | ○ | ○ | ○ |

| | | | |
|---|---|---|---|
| Footnotes are the same as in Table 2. | | | |

The results in Table 2 and 3 show that Examples 3 to 5, in which bis-ethylhexyloxyphenol methoxyphenyl triazine was added to the water phase, had enhanced ultraviolet protection effect relative to Comparative Example 5, in which bis-ethylhexyloxyphenol methoxyphenyl triazine was added to the oil phase and to Comparative Examples 2 to 4, in which other ultraviolet absorbent was used instead. Additionally, the results reveal that these compositions are superior in formulation stability and feeling on use as well.

## Claims

1. An oil-in-water sunscreen cosmetic composition, comprising:
(a) an aqueous dispersion having composite particles of bis-ethylhexyloxyphenol methoxyphenyl triazine and an organic polymer dispersed therein, wherein said composite particles are obtainable by emulsion-polymerization of a mixture of bis-ethylhexyloxyphenol methoxyphenyl triazine and an organic monomer having an ethylenically unsaturated bond dispersed in water;
(b) 0.01 to 5% by mass of one or two or more of crosslinked polyether-modified silicones and crosslinked alkyl-polyether-modified silicones;
(c) 0.01 to 30% by mass of a dimethicone having a viscosity of 10 mPa·s or less; and
(d) 0.01 to 80% by mass of water,
wherein the amount of bis-ethylhexyloxyphenol methoxyphenyl triazine in the cosmetic composition is 0.01 to 5% by mass.

2. The cosmetic composition according to claim 1, wherein the amount of bis-ethylhexyloxyphenol methoxyphenyl triazine is 0.01 to 3% by mass.

3. The cosmetic composition according to claim 1 or 2, further comprising methylene bisbenzotriazolyl tetramethylbutylphenol.

## Patentansprüche

1. Kosmetische Zusammensetzung in Form eines Öl-in-Wasser-Sonnenschutzmittels, umfassend:
(a) eine wässrige Dispersion, in welcher Partikel eines aus Bis-Ethylhexyloxyphenolmethoxyphenyltriazin und einem organischen Polymer gebildeten Verbundstoffes dispergiert sind, wobei die Partikel des Verbundstoffes durch Emulsionspolymerisation eines in Wasser dispergierten Gemisches aus Bis-Ethylhexyloxyphenolmethoxyphenyltriazin und einem eine ethylenisch ungesättigte Bindung aufweisenden organischen Monomer erhältlich sind;
(b) 0.01 bis 5 Masse% an einem oder zwei oder mehreren von vernetzten Polyether-modifizierten Silikonen und vernetzten Alkylpolyether-modifizierten Silikonen;
(c) 0.01 bis 30 Masse% an einem Dimethicon, welches eine Viskosität von 10 mPa·s oder weniger aufweist; und
(d) 0.01 bis 80 Masse% an Wasser,
wobei die Menge an Bis-Ethylhexyloxyphenolmethoxyphenyltriazin in der kosmetischen Zusammensetzung 0.01 bis 5 Masse% beträgt.

2. Kosmetische Zusammensetzung nach Anspruch 1, wobei die Menge an Bis-Ethylhexyloxyphenolmethoxyphenyltriazin 0.01 bis 3 Masse% beträgt.

3. Kosmetische Zusammensetzung nach Anspruch 1 oder 2, weiterhin umfassend Methylen-bisbenzotriazolyltetramethylbutylphenol.

## Revendications

1. Composition cosmétique d'écran solaire huile-dans-eau, comprenant :
(a) une dispersion aqueuse ayant des particules composites de bis-éthylhexyloxyphénol méthoxyphényl triazine et un polymère organique dispersé dans celle-ci, dans laquelle lesdites particules composites sont susceptibles d'être obtenues par polymérisation en émulsion d'un mélange de bis-éthylhexyloxyphénol méthoxyphényl triazine et d'un monomère organique ayant une liaison éthyléniquement insaturée dispersé dans de l'eau ;
(b) 0,01 à 5 % en masse d'une ou deux ou plus parmi les silicones modifiées par polyéther réticulées et les silicones modifiées par alkylpolyéther réticulées ;
(c) 0,01 à 30 % en masse d'une diméthicone ayant une viscosité de 10 mPa.s ou moins ; et
(d) 0,01 à 80 % en masse d'eau,
dans laquelle la quantité de bis-éthylhexyloxyphénol méthoxyphényl triazine dans la composition cosmétique est de 0,01 à 5 % en masse.

2. Composition cosmétique selon la revendication 1, dans laquelle la quantité de bis-éthylhexyloxyphénol méthoxyphényl triazine est de 0,01 à 3 % en masse.

3. Composition cosmétique selon la revendication 1 ou 2, comprenant en outre du méthylène bisbenzotriazolyl tétraméthylbutylphénol.
